# EUROPEAN PATENT SPECIFICATION

(11) **EP 2 555 640 B1**
(45) Date of publication and mention of the grant of the patent: **05.09.2018**
(21) Application number: 11764991.3
(22) Date of filing: 08.04.2011
(51) Int. Cl.: A23B 7/10, A23P 20/17, A23B 7/154, A23B 7/157, A23B 7/16, A23L 29/231, A23L 29/256, A23P 20/10

(54) **EDIBLE COATING COMPOSITION AND USES THEREOF**
VERZEHRBARE BESCHICHTUNGSZUSAMMENSETZUNG UND VERWENDUNGEN DAVON
COMPOSITION D'ENROBAGE COMESTIBLE ET SES UTILISATIONS

(30) Priority: 09.04.2010 US 322499 P
(43) Date of publication of application: 13.02.2013
(73) Proprietor: Fruitsymbiose Inc., St Nicolas, QC G7A 4X6 (CA)
(72) Inventor: GIRARD, Geneviève, Levis, Québec G6V 7A1 (CA)
(74) Representative: Kopff, Hélène
(86) International application number: PCT/CA2011/000392
(87) International publication number: WO 2011/123949

(56) References cited:
- WO-A1-94/02026
- WO-A1-94/02026
- WO-A1-96/01566
- WO-A1-99/07230
- WO-A2-98/42214
- WO-A2-2009/061221
- ES-B1- 2 138 559
- FR-A1- 2 821 245
- GB-A- 681 843
- GB-A- 681 843
- US-A- 4 624 856
- US-A- 5 552 166
- US-A- 6 159 512
- M.S. TAPIA ET AL: "Alginate- and Gellan-Based Edible Films for Probiotic Coatings on Fresh-Cut Fruits", JOURNAL OF FOOD SCIENCE, vol. 72, no. 4, 1 May 2007 (2007-05-01), pages E190-E196, XP055024511, ISSN: 0022-1147, DOI: 10.1111/j.1750-3841.2007.00318.x
- ROJAS-GRAU ET AL: "Alginate and gellan-based edible coatings as carriers of antibrowning agents applied on fresh-cut Fuji apples", FOOD HYDROCOLLOIDS, ELSEVIER BV, NL, vol. 21, no. 1, 1 January 2007 (2007-01-01), pages 118-127, XP005664095, ISSN: 0268-005X, DOI: 10.1016/J.FOODHYD.2006.03.001

## Description

### FIELD OF THE INVENTION

The present relates to a method for coating food products, more particularly, with an edible coating comprising a layer of a cross-linked polysaccharide.

### BACKGROUND OF THE INVENTION

Consumer awareness toward good eating habits created a need for ready-to-eat, practical and convenient fresh-cut fruits. This caused the market for fresh-cut fruit to exponentially increase over the last decade.

Because fresh fruits and vegetables consumed in urban areas are most of the time produced in remote regions and/or foreign countries, their distribution to consumers generally requires storage, handling and transport, which makes them prone to damage and spoilage. In order to maintain quality, shelf-life and safety of fresh fruits and vegetables, several handling and postharvest steps for improving or enhancing their protection have been considered. These steps include complete sanitation programs using chemical sanitizing agents, ozone or hot dips, refrigeration, controlled atmosphere, modified atmosphere packaging and controlled ripening.

Another technique known to increase shelf-life of fresh products includes coating fresh food product with an edible coating. Edible coatings create a physical barrier between the fruit or vegetable and the surrounding environment, and reduce ripening reactions such as color and aroma changes, desiccation and degradation of the product. Edible coatings are thus effective in creating a micro-environment for each individual fruit or part thereof, thereby retaining humidity and reducing respiration and oxidation and extending shelf life of the products.

More recently, edible coatings were also envisioned to serve as vehicles for adding functional ingredients to the fresh product. Known functional ingredients include antimicrobial agents (*e.g.* essential oils), antioxidants (*e.g.* organic acids), texture enhancers (*e.g.* glycerol) and nutraceuticals (*e.g.* probiotics, prebiotics and omega-3), which are embodied in the coating to promote health benefits and provide added nutritive value to the coated product.

Edible coatings known in the art include polysaccharide-, protein- and lipid-based edible coatings. Protein-based edible coatings typically include whey protein, soy protein, gluten, corn protein and/or sodium caseinate. While being efficient, the use of protein-based coating may be limited by current concerns with food allergies since many of the protein ingredients trigger allergic responses. Further, vegetarians and vegans may tend to avoid products coated with protein-based coating because they are derived from an animal source.

Lipid ingredients used for the production of edible coatings include shellac, beeswax, candelilla wax, carnauda wax and fatty acids. Again, some of the lipid-based coatings are from animal sources and tend to be avoided by vegetarians and vegans, which makes them unsuitable for coating products that are, at least partially, destined to this market segment.

Polysaccharides from plant, seaweed and/or bacteria origin have thus been studied for their jellification capacities. The polysaccharides most commonly used are cellulose derivatives, alginate, carrageenan, chitosan, pectin, starch derivates and other gums. Sodium alginate and carrageenan are both derived from seaweed whereas gellan is produced by a bacteria, *Sphingomonas elodea.*

While polysaccharide-based coatings avoid some of the drawbacks associated with protein- and/or lipid-based coatings, sodium alginate, carrageenan, gellan and other polysaccharide-based coating require a cross-linking agent to jellify. Cross-linking agents typically contain monovalent, divalent or trivalent cations and studies have reported the use CaCl₂ or KCI for this purpose. For example, the use of CaCl₂ to set a gel coating is described in US Patent No. 6,159,512. One of the major drawbacks of using these calcium and potassium salts is that they tend to create turbid solutions when dissolved in water and provide a bitter taste to the coated product, which is undesirable in many instances.

In US Patent No. 5,939,117, Chao et al. briefly describe coating avocado slices with sodium alginate followed by dipping in calcium ascorbate and then preserving the coated avocado in a relative humidity higher than 98%. Chao later stated in US Patent No. 5,925,395 that it is preferable to avoid the use of film forming agents, waxes, gums and polysaccharides such as alginates and carrageenans in vegetable preservatives since they affect the "mouth feel" of the cut vegetable and impart a waxy of slippery feel. In addition to provide unpleasant sensations to customers, such polysaccharide coatings tend to be good microbial culture mediums. Because fresh-cut fruits (e.g. pineapple slices, etc.) are more subject to microbial spoilage than whole fruits (e.g. grapes, apples, pears) and product deterioration and food safety issues are more likely to occur (Brecht 1995; Thunberg *et al.* 2002), polysaccharide-based edible coatings known in the art have therefore not all proven effective.

The following documents disclose further methods for preserving foods or stabilizing biological material.

WO96/01566A1 teaches a composition for preserving exposed underground plant structures which comprises an edible coating which acts as a carrier for an anti-browing agent and at least one anti-browing agent.

WO99/07230A1 discloses a method for preserving fresh fruit with fresh fruit preservatives which extend the shelf life of fresh fruits, particularly fresh cut fruits.

WO98/42214A2 discloses edible films and coatings suitable for use as moisture barriers. FR2821245A1 discloses a process for restructuring food, including meat products, fish products, fruits products, and cereals, either raw or cooked.

Tapia et al. (J. Food Science, 2005, Vol. 72, pages E190-E196) teaches alginate- and gellan-based edible films for probiotic coating on fresh-cut fruits and Rojas-Grau et al. (Food Hydrocolloids, 21 (2007) 118-127) teaches the same as carriers of antibrowning agents applied on fresh-cut Fuji apples.

WO94/02026A1 discloses a process for coating of candied foodstuff with an alginate gel or calcium or aluminium pectate film.

WO2009/061221A1 discloses a composition and method of manufacture including a substrate coated with a biopolymer and aqueous gel biological gel and subsequently coated with at least one desiccation agent.

It would thus be advantageous to be provided with an edible coating that addresses at least one of the above drawbacks.

### BRIEF SUMMARY OF THE INVENTION

A method for coating a food product with an edible coating is provided. This edible coating comprises a polysaccharide layer including at least one cross-linked polysaccharide. The at least one cross-linked polysaccharide is selected from the group consisting of carrageenan, gellan, alginate and pectin, and is cross-linked with a cross-linking agent which comprises calcium ascorbate. The method for coating a food product with an edible coating comprises:
(1) coating the food product with a polysaccharide solution to cover the food product, the polysaccharide solution including at least one polysaccharide selected from the group consisting of carrageenan, gellan, alginate and pectin;
(2) cross-linking said polysaccharide solution by immersing said food product in a cross-linking agent solution to obtain a polysaccharide layer covering said food product; said cross-linking agent solution comprising a calcium ascorbate solution, and
(3) reducing the moisture content of said polysaccharide layer to obtain said edible coating, using a drying process that minimizes thermoshocks to the food product, wherein the drying process is a process selected from the group consisting of a compression process, a desiccation process and a surface lyophilisation process and whereby the moisture content of the polysaccharide layer is reduced by 30% to 97% or, in other words, as such as the moisture content of the edible coating ranges from 3% to 70%.

In one aspect, the polysaccharide solution is an alginate solution, and preferably a sodium alginate solution. In this aspect, the polysaccharide solution preferably comprises between 0.1% (w/w) and 8% (w/w) sodium alginate, more preferably between 0.5% (w/w) and 4% (w/w) sodium alginate, even more preferably between 0.7% (w/w) and 3% (w/w) sodium alginate, and still even more preferably between 1% (w/w) and 2% (w/w) sodium alginate. In yet another aspect, the polysaccharide solution is a pectin solution, and preferably a pectin LM solution. In this aspect, the pectin LM solution preferably comprises between 2% (w/w) and 15% (w/w) pectin LM, and more preferably between 5% (w/w) and 10% (w/w) pectin LM.

In a further aspect, the polysaccharide solution comprises sodium alginate and pectin LM. In this aspect, the polysaccharide solution preferably comprises between 0.1% (w/w) to 3% (w/w) sodium alginate and between 0.1% (w/w) to 7% (w/w) pectin LM.

In still a further aspect, the calcium ascorbate solution comprises between 0.5% (w/w) and 34% (w/w) calcium ascorbate, more preferably between 1% (w/w) and 30% (w/w) calcium ascorbate, even more preferably between 13% (w/w) and 27% (w/w) calcium ascorbate and still even more preferably 15% (w/w) calcium ascorbate.

In an additional aspect, the polysaccharide solution further comprises a flavoring agent, where the flavoring agent is preferably a vanilla essence.

In yet an additional aspect, the polysaccharide solution further comprises an antimicrobial agent, and preferably vanillin or an essential oil.

In still an additional aspect, the polysaccharide solution further comprises an antioxidant agent. The antioxidant preferably comprises at least one of citric acid and ascorbic acid.

In another aspect, the polysaccharide solution further comprises a nutraceutical agent. The nutraceutical agent preferably includes at least one probiotic, where the probiotic is preferably selected from the group consisting of *Lactobacillus acidophilus, Lactobacillus casei* and, *Bifidobacterium lactis.*

In a further aspect, the polysaccharide solution further comprises an immune response enhancer, and preferably a yeast gluco polysaccharide.

In yet a further aspect, the polysaccharide solution further comprises at least one element selected from the group consisting of a coloring agent, a protein, an amino acid and a vitamin.

In another aspect, the step of immersing the food product in the cross-linking agent solution for a period of time to obtain the polysaccharide layer ranges from 1 second to 15 minutes, preferably from 10 seconds to 10 minutes, and more preferably from 10 seconds to 4 minutes.

In a further aspect, the polysaccharide solution and the cross-linking agent solution have a temperature below 37 Celsius degrees, and preferably have a temperature ranging from - 5 Celsius degrees to 20 Celsius degrees, and more preferably a temperature ranging from 4 Celsius degrees to 10 Celsius degrees, and even more preferably a temperature ranging from 4 Celsius degrees to 7 Celsius degrees.

In another aspect, the edible coating has a pH above 3, preferably a pH ranging between 4 and 9, and more preferably a pH ranging between 5 and 8.

In a further aspect, the method further comprises a step of sprinkling a food additive on a surface of the edible coating. Preferably, the food additive comprises granules of at least one dried fruit, and more preferably at least one dried fruit selected from the group consisting of a dried apple, a dried strawberry and a dried raspberry.

The resulting edible coating is used to extend a shelf-life of a perishable food product. In this embodiment, the food product is preferably at least one of a fruit and a vegetable. The method may involve grouping the food products to form clusters for obtaining clusters of food products.

In this embodiment, the method comprises: (1) coating the food product with a polysaccharide solution to substantially cover the food product, the polysaccharide solution including at least one polysaccharide selected from the group consisting of carrageenan, gellan, alginate and pectin; (2) grouping the food products to form clusters thereof; (3) cross-linking the polysaccharide solution by immersing the food product in a cross-linking agent solution to obtain a polysaccharide layer substantially covering the food product; and (4) reducing the moisture content of the polysaccharide layer to obtain the edible coating as detailed in present claim 1. In another embodiment, the method comprises grouping food products to form clusters thereof prior to coating with the polysaccharide solution. It is described a food product comprising the edible coating described hereinabove. The food product may further comprise a food additive sprinkled on the surface of the edible coating. The food additive preferably comprises granules of at least one dried fruit, and more preferably at least one dried fruit is selected from the group consisting of a dried apple, a dried strawberry and a dried raspberry.

It is further described a kit for a snack. In this embodiment, which does not form part of the invention, the snack kit comprises a package of a food product, the food comprising the edible coating described hereinabove; and a package of a food additive suitable for being sprinkle on a surface of said edible coating.

The food additive of the snack kit may comprise granules of at least one dried fruit, and more preferably at least one dried fruit is selected from the group consisting of a dried apple, a dried strawberry and a dried raspberry.

The snack kit may further comprise a tool for allowing a user to eat the food product, where the tool is preferably selected from the group consisting of a fork and a toothpick.

### BRIEF DESCRIPTION OF THE FIGURES

In order that the invention may be readily understood, examples, which do not form part of the invention, are illustrated in the accompanying drawings.
**Figure 1** shows the difference in appearance between uncoated and coated grapes after 21 days of storage.
**Figure 2** shows the difference in appearance between uncoated (control) and coated pineapple tidbits after storage at 4°C.
**Figure 3** shows the difference in appearance between uncoated (control) and coated blueberries after storage at 4°C.
**Figure 4** shows the formation of lowbush blueberries clusters.

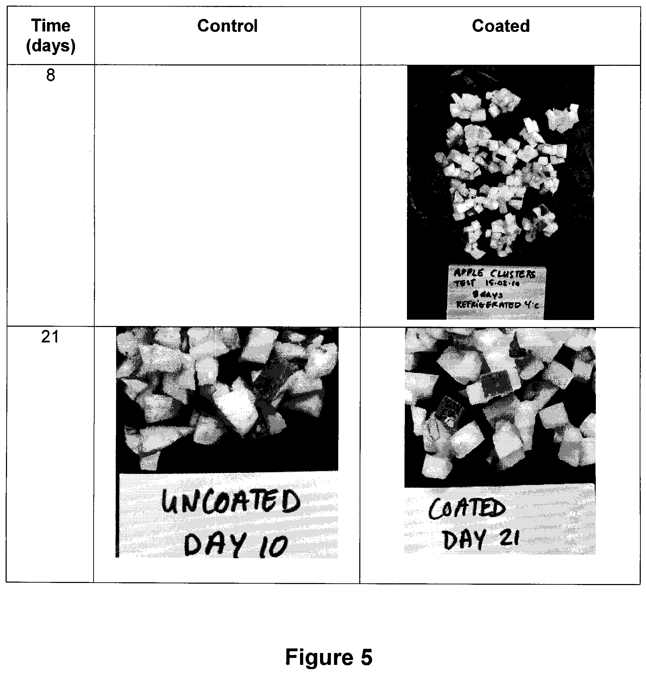

Figure 5 shows the difference in appearance between uncoated (control) and coated apple clusters after storage at 4°C.
**Figure 6** shows the difference in appearance between uncoated (control) and coated vegetables of a first mix of fresh cut vegetables after storage at 4°C.
**Figure 7** shows the difference in appearance between uncoated (control) and coated vegetables of a second mix of fresh cut vegetables after storage at 4°C.
**Figure 8** shows the difference in appearance between uncoated (control) and coated vegetables of a third mix of fresh cut vegetables after storage at 4°C.

Further details of the invention and its advantages will be apparent from the detailed description included below.

### DETAILED DESCRIPTION OF THE INVENTION

In the following description of the embodiments, references to the accompanying drawings are by way of illustration of examples, which do not form part of the invention. According to one embodiment, a method for coating a food product with an edible coating for a food product is provided. The edible coating is typically used for coating a perishable food product such as a fruit or a vegetable. Exemplary food products for use with the edible coating include, but are not limited to, whole and fresh cut fruits such as strawberries, grapes, blueberries, mangoes, papayas, apples, kiwis, cantaloupes, pineapples, honeydew melons, watermelons, and whole and fresh cut vegetables, such as bell peppers, carrots, turnips, onions (*e.g.* red and yellow onions), celery, leeks, broccolis, cauliflowers, potatoes, sweet potatoes, cabbages, zucchinis and the like. A person skilled in the art will appreciate that the edible coating may find use with any other product intended for animal or human consumption. For instance, the edible coating could be used for coating meat or fish products, as well as veggie meals such as veggie patties.

The edible coating is used to extend or prolong the shelf-life of fruits and vegetables. The terms "extending shelf-life", "prolonging shelf-life" and similar terms shall be interpreted broadly so as to include any gain in product conservation. This would include, for instance maintaining or preserving, at least partially, one or several of the appearance (*e.g.* color), texture or taste, or reducing desiccation (*i.e.* juice losses) of the product.

The edible coating comprises polysaccharide layer including at least one cross-linked polysaccharide selected from the group consisting of carrageenan, gellan, alginate and pectin, and has been cross-linked using a cross-linking agent.

In one embodiment, the cross-linked polysaccharide is alginate, and preferably sodium alginate. As it will become apparent below, experiments have shown that sodium alginate is capable of forming thin gels that are firm or very firm and yet be easy to masticate. Alternatively, the cross-linked polysaccharide is pectin, and more preferably pectin LM. The tests carried out with the different food products and the different polysaccharide/cross-linking agent combinations showed that pectin displayed better coating properties on food products having higher contents of juices or syrups (*e.g.* fruit salads) than sodium alginate, carrageenan and gellan (see Example 1 below). Accordingly, in instances where the food product to be coated is prone to exude substantial amounts of juice, the use of pectin would be preferred.

In some other instances, it may be desirable to use an edible coating comprising a combination of cross-linked polysaccharides, such as, for instance, a combination of sodium alginate and pectin LM. A person skilled in the art would nevertheless recognize that any combination of sodium alginate, pectin, carrageenan and gellan may work. The cross-linking agent is calcium ascorbate. The use of calcium ascorbate is desirable because this cross-linking agent tends to avoid the off-flavor, bitter taste, salty taste and/or chlorine taste associated with calcium and potassium sources known in the art (*e.g.* CaCl₂ or KCI), or with calcium lactate and calcium citrate, as described below in Example 1. In the present specification, the term "off-flavor" is used to describe a flavor (and an odor) generally associated with the degradation of a perishable food product. Accordingly, the term "off-flavor" as intended herein excludes a flavor or an odor conferred to the edible coating by the presence of an additional ingredient such as, for example, a probiotic, as it will become apparent below. Further, ascorbate is an ion of ascorbic acid (*i.e.* Vitamin C) and thus, the use of calcium ascorbate as cross-linking agent confers antioxidant properties to the edible coating, which may also make its use desirable. The obtained edible coating has a moisture content ranging from 3% to 70%. In other words, the moisture content of the cross-linked polysaccharide layer is reduced by 30% to 97% during a drying step, as it will become apparent below. The reduction of the moisture content of the edible coating matrix makes it less susceptible to microbial proliferation since the water is sequestered in the polysaccharide matrix, which in turn tends to expand the shelf-life of the coated food product. Further, the reduction of the moisture content tends to minimize the unpleasant mouth feel generally associated with the edible coatings of the prior art.

In one embodiment, the edible coating may further comprise an antimicrobial agent. For example, the use of an antimicrobial agent may be beneficial to further enhance the conservation properties of the edible coating. In one example, the use of vanillin as microbial agent is desirable because vanillin also contribute to mask the taste associated with some polysaccharides (*e.g.* the very mild algae taste associated with sodium alginate) or other elements that may be added to the edible coating (*e.g.* probiotics), and also enhance sweetness of products such as fruits. A person skilled in the art will appreciate that any other antimicrobial agent suitable for consumption may be used to replace, or in combination with vanillin. For example, one may opt for using essential oils, such as citrus essential oil, which are also known for their antimicrobial properties.

In a further embodiment, the obtained edible coating may also comprise an antioxidant agent, such as, for example, citric acid, ascorbic acid or a combination thereof. These antioxidants are known to have anti-browning properties. However, because these antioxidants also contribute to the cross-linking of polysaccharides, their concentration in the edible coating should not significantly lower its pH since, as it will become apparent in the examples below, a low pH may cause a premature jellification or cross-linking of the polysaccharide during the coating process, which in turn affect the uniformity of the coating on the food product. Accordingly, in one embodiment, the pH of the edible coating is above 3, and preferably ranges between 4 and 9, and more preferably ranges between 5 and 8.

Other ingredients such as a nutraceutical agent or an immune response enhancer can also be added to provide additional properties to the coating. The nutraceutical agent typically comprises at least one probiotic, examples of which include *Lactobacillus acidophilus, Lactobacillus casei* and, *Bifidobacterium lactic.* An example of immune response enhancer includes a yeast gluco polysaccharide, such as, for instance, Wellmune WGP®. A person skilled in the art will appreciate that many other functional ingredients can be added to the polysaccharide coating described herein. For instance a flavoring agent such as a vanilla essence can be used to provide a sweet taste to the coating. Alternatively, the edible coating could comprise at least one element selected from the group consisting of a coloring agent, a protein, an amino acid and a vitamin. In examples 1 to 7, which do not form part of the invention, edible coating compositions with different polysaccharide solutions and cross-linking agent solutions were tested. In one example, the polysaccharide solution comprises sodium alginate. The polysaccharide solution typically comprises between 0.1% (w/w) and 8% (w/w) sodium alginate, and more typically between between 0.5% (w/w) and 4% (w/w) sodium alginate, even more typically between 0.7% (w/w) and 3% (w/w) sodium alginate, and further even more typically between 0.7% and 2% sodium alginate. A person skilled in the art will appreciate that the concentration of polysaccharide used in solution may be selected based on the capacity to uniformly and rapidly coat the surface of the products, without compromising the ability to form a gel having a proper firmness.

Alternatively, the polysaccharide solution may comprise pectin, and more typically pectin LM. A person skilled in the art will appreciate that the pectin concentration in the solution used for the coating process may vary. Typically, the polysaccharide solution comprises between 2% (w/w) to 15% (w/w) pectin LM, and more typically between 5% (w/w) to 10% (w/w) pectin LM. Again, the tests carried out with the different food products and the different polysaccharide/cross-linking agent combinations showed that pectin LM would be preferable where the food product to be coated is prone to exude substantial amounts of juice, the use of pectin would be desirable.

In another example, the polysaccharide solution may comprise a combination of sodium alginate and pectin LM. In such a case, the polysaccharide solution would typically comprise between 0.1% (w/w) to 3% (w/w) sodium alginate and between 0.1% (w/w) to 7% (w/w) pectin LM. A person skilled in the art would acknowledge that many combinations of sodium alginate, pectin, carrageenan and gellan are possible, including a combination of at least one of those with other polysaccharides.

Coating the food product with the polysaccharide solution is carried out by immersing the food product in the polysaccharide solution. A person skilled in the art will appreciate that the immersion time required for covering the food product with the polysaccharide solution will depend upon the consistency of the solution and the size of the fruit.

To cross-link the polysaccharide solution to obtain a gel, the food product coated with the polysaccharide solution is immersed in the cross-linking agent solution. In one example, the cross-linking agent solution comprises between 0.5% (w/w) and 34% (w/w) calcium ascorbate, and typically between 1% (w/w) and 30% (w/w) calcium, and more typically between 13% (w/w) and 27% (w/w) calcium ascorbate, and even more typically 15% (w/w) calcium ascorbate. A person skilled in the art will appreciate that concentrations above34% (w/w) would also work. For instance, a calcium ascorbate solution at saturation may be used. Saturation of a calcium ascobrate solution typically occurs at a calcium ascorbate concentration of 50% (w/w), but the person skilled in the art will appreciated that concentration at saturation will vary based on the temperature of the solution.

Immersion of the food product in the cross-linking agent solution allows a simultaneous contact of all surfaces of the food product coated with the cross-linking agent solution and thus a uniform jellification or cross-linking of the polysaccharide on the food product. A person skilled in the art will appreciate that the immersion time for allowing cross-linking of the polysaccharide solution will be based upon the concentration of cross-linking agent in the solutions and the thickness of the polysaccharide layer to be cross-linked (*i.e.* generally, the thicker is the layer of polysaccharide to be cross-linked, the longer is the immersion time) . For example, an immersion time of 15-20 seconds in a solution comprising 15% calcium ascorbate would be sufficient to allow proper gel formation while the use of a 0.5% calcium ascorbate solution would require an immersion time of 5 to 8 minutes. Accordingly, the food product is typically immersed in the cross-linking agent solution for a period of time ranging from 1 second to 15 minutes, and more typically for a period of time ranging from 10 seconds to 10 minutes, and even more typically from 10 seconds to 4 minutes.

As it will be appreciated by a person skilled in the art, the short period of time required for jellification or cross-linking of the polysaccharide makes it suitable for rapidly coating food products and is advantageous for coating large volumes of food products such as, for instance, on an industrial scale.

Once the polysaccharide solution has been cross-linked with the cross-linking agent solution, the food product is coated with a generally uniform polysaccharide layer. However, because of it high moisture content, polysaccharide layer would tend to affect the "mouth feel" of the coated food product and to provide unpleasant sensations to customers. Further, the polysaccharide layer would be more subject to microbial proliferation and would reduce the effectiveness of the coating. Therefore, the moisture content of the polysaccharide layer is reduced to obtain the edible coating. A person skilled in the art will appreciate that it is preferable to maintain the temperature of the food product at a low temperature (*e.g.* at 4°C) during the drying process since event a slight increase of the food product temperature (*i.e.* an increase of 2°C) is susceptible to trigger enzyme activity and thus to affect its conservation during the coating process. Therefore, the moisture content is reduced by drying the food product by using a drying process that minimize thermoshocks to the food product. The drying process is a process selected from the group consisting of a compression process, a desiccation process and a surface lyophilisation process. The moisture content of the polysaccharide layer is reduced by 30% to 97% or, in other words, such that the moisture content of the edible coating ranges from 3% to 70%. When the food product coated is perishable food product, it is desirable to minimize their exposure to relatively high temperature during the coating process. Accordingly, in one embodiment, the polysaccharide solution and the cross-linking agent solutions have a temperature below 37 Celsius degrees during the coating process, and preferably a temperature ranging from -5 Celsius degrees and 20 Celsius degrees, and more preferably between 4 Celsius degrees and 10 Celsius degrees, and even more preferably between 4 Celsius degrees and 7 Celsius degrees. Similarly, the drying step of the polysaccharide layer to obtain the edible coating is carried out by drying the food product by using a drying process that minimize thermoshocks to the food product.

As stated above, the obtained edible coating may further comprise a flavoring agent, an antimicrobial agent, an antioxidant agent, a nutraceutical agent, an immune response enhancer, a coloring agent, a protein, an amino acid, a vitamin or others food additives. In one embodiment, these agents are added in the polysaccharide solution prior to the cross-linking step. A person skilled in the art would appreciate that they could alternatively be added to the cross-linking agent solution. As stated above however, the addition of components such as antioxidants to the polysacharride solution or to the cross-linking solution may may cause a premature jellification or cross-linking of the polysaccharide during the coating process, which in turn affect the uniformity of the coating on the food product. Accordingly, in one embodiment, the pH of the polysaccharide solution and the cross-linking agent solution is maintained above 3, and preferably ranges between 4 and 9, and more preferably ranges between 5 and 8.

A person skilled in the art will appreciate that the properties of the edible coating makes it suitable for preparing ready to consume food products such as fruit or vegetable snack. Accordingly, it is described a snack comprising a food product coated with the edible coating described above. In one example, the snack kit comprises a first package comprising the coated food product and a second package comprising a food additive capable of being sprinkle by the consumer on the food product (*i.e.* on the surface of the edible coating) at the time of consumption. In one non limitative example, the food additive comprises a powder or granules of at least one dried fruit, and typically the at least one dried fruit is selected from the group consisting of a dried apple, a dried strawberry and a dried raspberry. In one example, the food additive comprises a mixture of dried fruits. A person skilled in the art would appreciate that dried fruits can be obtained according to different methods, such as, for instance, drum drying and freeze drying. A person skilled in the art would also appreciate that any other food additive could be used, such as for instance sugar, cinnamon, condiments and the like.

In one example, the package of food additive is wrapped with the package of food product using a plastic membrane. Alternatively, the food additive package could be placed inside the package of food product prior to sealing the same. Typically, the first package (*i.e.* the package of food product) is a plastic tray heat sealed with a microperforated membrane, as described in the examples below, while the second package (*i.e.* the food additive package) is a plastic pouch or bag impermeable to humidity.

In a further example, the snack kit may further comprise a tool for allowing a user to eat said food product. Examples of such tools include a fork and a toothpick. Typically, the tool is packaged inside the first package or is wrapped with the first and second packages using a plastic membrane. A person skilled in the art will appreciate that many packaging possibilities exist for packaging a snack and that the examples herein provide are not exhaustive. As mentioned above, in examples 1 to 7, which do not form part of the invention, edible coating compositions with different polysaccharide solutions and cross-linking agent solutions were tested.

### EXAMPLE 1

### EDIBLE COATING COMPOSITIONS

A first selection was made based on the known characteristics of the different agents. Protein-based compositions were avoided because of current concerns with food allergens and because many of these ingredients are isolated from animal sources. Further to the screening based on the known characteristic of each component, polysaccharide compositions were selected.

To determine which polysaccharide would display the best properties using fewer components, several compositions were tested for their gelling properties, including those listed in Table 1 below.

**Table 1: Edible coating compositions**

| **No.** | **Jellifying agent** | **Cross-linking Agent** | **Other (with jellifying agent)** |
|---|---|---|---|
| 1. | Carrageenan (1% to 2.5%) | Calcium Ascorbate (15% w/w) | Vanilla essence (0.1%) |
| 2. | Gellan (0.5% to 2%) | Calcium Ascorbate (15% w/w) | Vanilla essence (0.1%) |
| 3. | -Carrageenan (0.1 to 0.5%) | Calcium Ascorbate (15% w/w) | Vanilla essence (0.1%) |
| | -Gellan (0.1 to 0,3%) | | |
| | -Sodium alginate (0.7 to 1.5%) | | |
| 4. | Sodium alginate (1%) | Calcium Ascorbate (15% w/w) | Vanilla essence (0.1%) |
| 5. | Sodium alginate (1.5%) | Calcium Ascorbate (15% w/w) | Vanilla essence (0.1%) |
| 6. | Sodium alginate (1.5%) | Calcium Lactate (15% w/w) | Vanilla essence (0.1%) |
| 7. | Sodium alginate (1.5%) | Calcium Citrate (15% w/w) | Vanilla essence (0.1%) |
| 8. | Sodium alginate (1.5%) | Calcium Ascorbate (15% w/w) | -Vanilla essence (0.1%) |
| | | | -Citric acid (1%) |
| 9. | Sodium alginate (1.5%) | Calcium Ascorbate (15% w/w) | -Vanilla essence (0.1%) -ascorbic acid (1%) |
| 10. | Sodium alginate (1.5%) | Calcium Ascorbate (15% w/w) | -Vanilla essence (0.1%) |
| | | | - Wellmune WGP® (0.5 to 1%) |
| 11. | Sodium alginate (1.5%) | Calcium Ascorbate (1 to 15% w/w) | -Vanilla essence (0.1%) |
| 12. | Sodium alginate (1.5%) | Calcium Ascorbate (15% w/w) | -Vanilla essence (0.1%) |
| | | | *-Lactobacillus acidophilus* (probiotic) (2%) |
| 13. | Sodium alginate (1.5%) | Calcium Ascorbate (15% w/w) | -Vanilla essence (0.1%) |
| | | | *-Lactobacillus casei* (probiotic) (2%) |
| 14. | Sodium alginate (1.5%) | Calcium Ascorbate (15% w/w) | -Vanilla essence (0.1%) |
| | | | *-Bifidobacterium bifidum* (probiotic) (2%) |
| 15. | Sodium alginate (1.5%) | Calcium Ascorbate (15% w*w) | |
| 16. | Sodium alginate (1.5%) | Calcium Ascorbate (15% w/w) | -Vanilla essence (0.1%) |
| | | | -Citric acid (1%) |
| | | | -Ascorbic acid (1%) |
| 17. | Pectin (5% to 10%) | Calcium Ascorbate (15% w/w) | Vanilla essence (0.1%) |

Sodium alginate, carrageenan, gellan and/or pectin were solubilized in stainless steel tanks with tap water at 50°C. The concentrations jellifying agents were based on the type of fruit to be coated and typically ranged from 1% to 1.5% w/w for sodium alginate, carrageenan and gellan and from about 5% to about 10% for pectin. For example, a lower sodium alginate concentration (of 1% w/w for acid fruits) was used with fresh-cut fruits such as pineapple tidbit since sodium alginate jellifies at acid pH and fresh-cut fruits tend to release acidic juice. The juice released tends to acidify the solution and contributes to unwanted, premature gel formation. Accordingly, a gel having expected characteristics (*i.e.* firmness and resistance) can be obtained using a lower sodium alginate concentration.

Upon solubilization, the sodium alginate, carrageenan, gellan and/or pectin solutions were refrigerated at 10°C and kept at this temperature throughout the coating of the fruits. As described in Table 1, natural vanilla essence (0.1%) and/or other agents were added in some instances. The purpose of the natural vanilla extract was to mask the mild algae taste associated with the use of sodium alginate, to enhance the natural sweetness taste of the fruits (sweetness enhancer), to act as an antimicrobial agent and to provide a new taste to the consumers.

Sodium alginate, carrageenan, gellan and/or pectin solutions all form gels upon the cross-linking action of the divalent cations. Hence, calcium ascorbate, calcium lactate and calcium citrate solutions were prepared by solubilizing powders (H&A Canadian industrial inc., Ontario, Canada) in tap water. The concentration of the respective solutions is described above in Table 1. Cross-linking agents solutions were kept between 4°C and 10°C for the duration of the coating process.

To assess the various characteristics of gel formation and the polyvalence of the different gels, two fruit models were tested and several fruit types were assessed in each model: strawberries, grapes, blueberries and blackberries were used as whole fruits models while, papayas, apples, kiwis, cantaloupes, pineapples, melon dews and watermelons were used as fresh-cut models.

The various coatings tested were assessed for their capability to retain the fruit's inherent juices, for their transparency, flexibility, taste and texture, ability to cover the whole fruit and mechanical resistance through storage time and handling.

### Results

The results of the analysis conducted on the various edible-coating tested are described in Table 2 below.

**Table 2: Characteristics of the edible coatings tested.**

| **No.** | **Gel characteristics** |
|---|---|
| 1. | Weak gel, brittle |
| 2. | Weak gel, brittle, cloudy, no off-flavor |
| 3. | Different mixtures comprising varying concentrations of sodium alginate, carrageenan and gellan were tested to assess whether synergistic effects may occur. Under the tested conditions, the presence of carrageenan and/or gellan did not significantly strengthened the gel structure. Under some conditions, the presence of carrageenan and/or gellan even appeared to weaken the gel, which may be desirable for some applications. |
| 4. | Firm gel, clear, elastic, no off-flavor |
| 5. | Very firm gel, clear, elastic, no off-flavor |
| 6. | Very firm gel, clear, elastic, off-flavor (attributable to calcium source) |
| 7. | Very firm gel, clear, elastic, off-flavor (attributable to calcium source) |
| 8. | Very firm gel, clear, elastic, low pH, no off-flavor |
| 9. | Very firm gel, clear, elastic, low pH, no off-flavor |
| 10. | Very firm gel, elastic, a little cloudy, Wellmune WGP® taste. |
| 11. | Different concentrations of calcium ascorbate were tested. Best gel firmness in the least amount of time is what conditioned the choice of the calcium ascorbate concentration. The setting of the gel by a crosslink agent is time dependant. In an industrial context, the time needed to set the gel must be short. We set that a 12-15 seconds gelling time was sufficient to obtain firm gels. Optimizing of the concentration was also related to the cost of ingredients. This is why a 15% concentration was chosen. |
| 12. | Firm gel, elastic, cloudy, mild yogurt odor and taste, no off-flavor |
| 13. | Firm gel, elastic, cloudy, mild yogurt odor and taste, no off-flavor |
| 14. | Firm gel, elastic, cloudy, mild yogurt odor and taste |
| 15. | Firm gel, very mild algae taste, no off-flavor. |
| 16. | Sodium alginate jellified before contact with the cross-linking agent (calcium ascorbate). The premature jellification is due to low pH from the added antioxidants (citric acid and ascorbic acid). While these antioxidants would normally contribute to extend fruit shelf-life, the pH is too low making their use unsuitable for proper gel formation. |
| 17. | Firm gel, brittle, no off-flavor. Pectin tends to provide better results (e.g. texture and taste) than sodium alginate with fruits in syrup. |

These results showed that sodium alginate as sole polysaccharide source, in combination with calcium ascorbate, provides the best gel texture, resistance and a complete fruit coverage, without off-flavor. Further, the combination of sodium alginate and calcium ascorbate provided the best results with the broader variety of fruits or, in other words, appeared to be the more polyvalent combinations. Pectin showed better results than sodium alginate with fruits in syrup such as, for instance, fruit salad. Carrageenan and gellan provided weaker yet acceptable gel properties.

Vanilla extract was effective to mask the mild algae taste associated with the use of sodium alginate and to enhance the natural sweetness taste of the fruits.

Antioxidants (citric and ascorbic acids) may have a positive effect on the gel resistance. However, they must be used at low concentrations to avoid compromising the setting of the gel by the cross-linking agent.

Probiotic bacterias added to the edible coating composition showed a good survival rate for the whole duration of the experiments, which corresponds to the products' shelf-life. The addition of probiotic bacterias brought mild yogurt odor and taste to the composition.

To test the effectiveness of this composition in preserving or extending the shelf-life of fresh products, the composition was tested on four (5) fruit models, namely table grapes (Example 2), pineapple tidbits (Example 3), lowbush blueberries clusters (Example 5) and apple clusters (Example 6).

### EXAMPLE 2

### EFFECTIVENESS OF THE SODIUM ALGINATE COMPOSITION ON FRESH FRUITS HAVING PEEL

### Grapes supply

Fresh table grapes were used as a model to fresh fruits that conserve their natural peel such as blueberries, cherries, and the like. Grapes (Flames, Crimson or Red Globe cultivars) were cultivated in California, Mexico or Chile and were purchased from Margi-Fruit, Quebec, Canada. Grapes were imported from California to Canada in monitored refrigerated containers. Transportation took approximately 5 to 7 days.

### Table grape preparation

Grapes were kept in their original package (regular plastic bag found in retail markets) until the experiment.

Grapes were divided into first control grapes (kept in their original package for the whole duration of the experiment), uncoated control grapes and coated grapes. For uncoated control grapes and coated grapes, whole table grape clusters were immersed in a peracetic acid and hydrogen peroxide solution kept at 4°C Chinook® (Sani Marc, Quebec, Canada) or Tsunami 100®, (Ecolab, Quebec, Canada) for 15 seconds. While in immersion, grape clusters were gently agitated to ensure thorough washing. Grapes were detached from the stems and sorted out to discard the damaged and/or rotten grapes.

Individual grapes were further sanitized again in a different a peroxyacetic acid and hydrogen peroxide solution kept at 4°C (Chinook® or Tsunami 100®) for 15 seconds, after, which they were air dried for 3 minutes using hair dryer (20-24 km/hr) on a conveyor belt.

### Preparation of sodium alginate coating

24 hours before the actual coating of the fruits, sodium alginate (1.5% w/w), was solubilized in a tank with tap water at 50°C. Natural vanilla essence was added to the sodium alginate solution in a concentration of 0.1% (Ingredient #33282, David Michael Ingredients, AZ, USA). The final concentration of sodium alginate used for table grapes was 1.5% w/w since the gel formed by the crosslinking action of calcium ascorbate appeared to be optimal at this concentration. The sodium alginate/vanilla solution was refrigerated a 10°C and kept at this temperature until before the coating of the fruits.

Meanwhile, a solution of 15% w/w of calcium ascorbate (H&A Canada Industrial inc., Ontario, Canada) was prepared by dissolving calcium ascorbate in tap water and kept between 4°C and 7°C for the duration of the experiments.

### Coating of the grapes with the sodium alginate gel

Grapes were individually enrobed in the in the sodium alginate solution for 10 seconds. Grapes were held on a conveyor belt and excess solution was drained for 10 seconds. Grapes were then immersed individually in the calcium ascorbate solution (15% w/w) for 15 to 20 seconds and then held on a conveyor belt to allow excess solution to drain. Grapes were later air dried for 6 min with a domestic hair dryer (20-24 km/hr) on a conveyor belt.

### Coated grapes conditioning

Uncoated and coated grapes were packaged (first control grapes remained in their original package). The packaging was designed for the coated fruits aims to prevent produce dehydration and maintain optimal CO₂/O₂ ratios inside the packaging to respect produce respiration (controlled atmosphere). Accordingly, grapes were packaged in PETE 3.7 oz plastic trays. The trays were heat-sealed with a microperforated PET based film (Ultraperf, Quebec, Canada). The permeability of the film allowed for a maximum buildup of 15% CO₂ and 5% O₂. The headspace within the trays accounted for 50% of the total volume. All sealed containers were refrigerated at 4°C, in the dark, for 21 days.

### Results

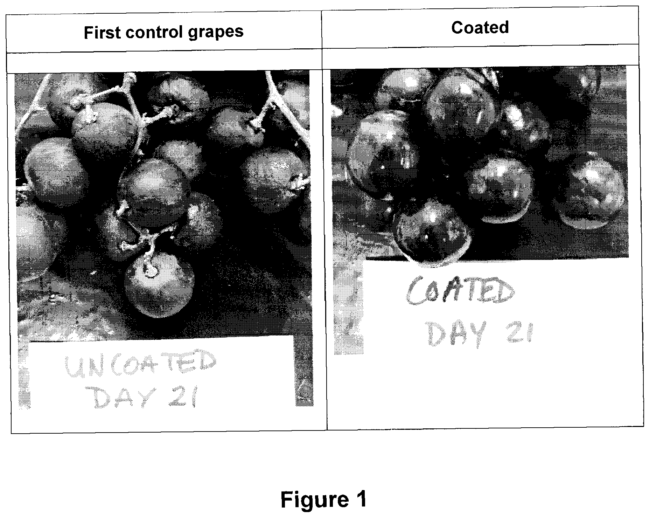

The main effect of the edible coating and packaging on whole table grapes was a significant reduction in desiccation after 21 days, as best shown in Figure 1 below. The edible coating and packaging also contributed to preserve the original color and texture of the test grapes throughout the storage time. The natural vanilla extract present in the edible coating enhanced the grape's sweetness.

The sensory attributes of appearance, texture and taste were evaluated on a scale of 1 to 5, where a ranking of 1 suggests poor quality and unacceptability whereas a ranking of 5 suggests excellent quality and acceptability. These three quality attributes were evaluated for the grapes throughout the storage period of 21 days.

First control grapes (i.e. kept in the original commercial package) were dehydrated and had off-flavors coming from the refrigerated atmosphere after 21 days, ranking 1 (i.e. the lowest possible rank) after 21 days. At 21 day, the control, uncoated grapes scored 3 for appearance, texture and taste. The PETE packaging heat-sealed with micro-perforated PET film thus appeared to significantly contribute to increase the shelf-life of grapes. The inherent oxygen and carbon dioxide ratio inside each package thus appeared to reduce microbial spoilage. The coated grapes displayed the best characteristics, scoring a 5 for appearance, texture and taste. The coating, in combination with packaging, thus contributed to maintain the initial quality of the grapes throughout 21 days and notably increased the shelf-life of grapes.

### EXAMPLE 3

### EFFECTIVENESS OF THE EDIBLE COATING WITH FRESH-CUT FRUITS - PINEAPPLE

### Fresh fruit supply

Pineapple tidbits were used as model for fresh-cut fruits for which the interior of the fruit is exposed to environmental conditions. As such, pineapple results obtained with pineapple tidbits may be expanded to other cut fruits such as apples, papaya, kiwi, pomegranates, clementines, pears, melon dew, cantaloupe and the like. Pineapples were cultivated in Costa Rica and were purchased from Margi-Fruits, Quebec, Canada. Pineapples were carried from Costa Rica to Canada in monitored refrigerated containers in 5 to 10 days.

### Preparation of pineapple tidbits

Whole pineapples were soaked in a peroxyacetic acid and hydrogen peroxide solution (Chinook® or Tsunami 100®) for 15 seconds. The temperature of the solution was kept at 4°C. While in the solution, the pineapples were lightly brushed using a manual hand brush to cleanse them thoroughly. On a sanitized surface, the pineapples were peeled and cored. The pineapple segments obtained were sanitized in a new solution of peracetic acid and hydrogen peroxide. Still on a sanitized surface, the pineapple segments were then cut into tidbits using a sanitized knife.

Pineapple tidbits were held in a strainer to remove excess juice until experiments.

### Preparation of sodium alginate coating

Sodium alginate (1.0% w/w) and calcium ascorbate (15% w/w, H&A Canada Industrial inc., Ontario, Canada) solutions were prepared as described above. Natural vanilla essence was added to the sodium alginate solution in a concentration of 0.1% (Ingredient #33282, David Michael Ingredients,PA, USA). The sodium alginate/vanilla solution was refrigerated at 10°C and kept at this temperature until before the coating of the fruits. The calcium ascorbate solution was kept between 4°C and 7°C for the duration of the experiments.

### Coating of the pineapple tidbits with the sodium alginate gel

Pineapple tidbits were divided into control tidbits and test tidbits, where control tidbits remained uncoated.

Test tidbits were individually enrobed in the in the sodium alginate solution. Test pineapple tidbits were held on a conveyor belt and excess solution was drained for 10 seconds. Tidbits were then immersed individually in the calcium ascorbate solution for 15 to 20 seconds and then again held on a conveyor belt to allow excess solution to drain. Pineapple tidbits were later air dried for 8 min with a domestic hair dryer (20-24 km/hr) on a conveyor belt.

### Coated pineapple tidbits conditioning

Control and test pineapple tidbits were packaged in PETE 3.7 oz plastic trays heat-sealed with a microperforated PET based film, as described above. The headspace within the trays accounted for 50% of the total volume. All sealed containers were refrigerated at 4°C, in the dark, for 15 days.

### Results

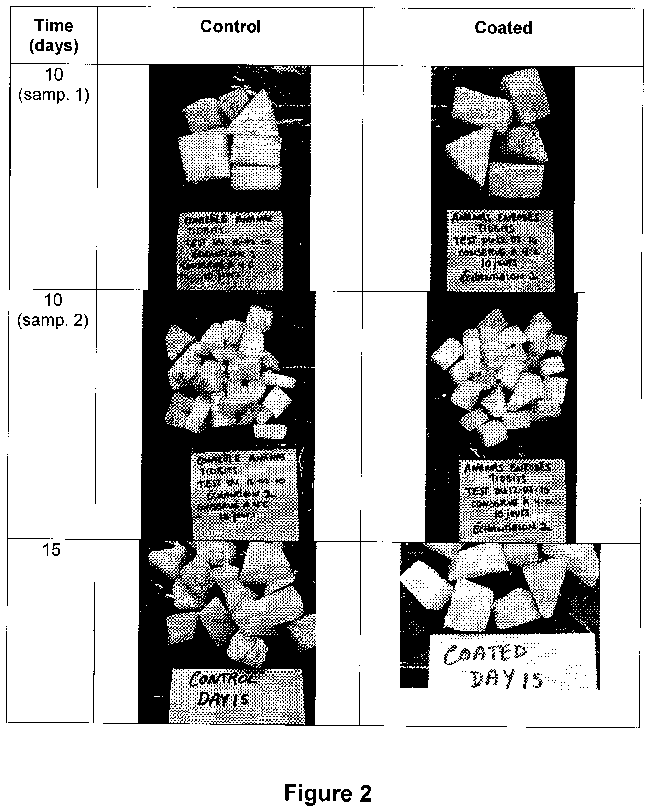

The most noticeable effect of the edible coating on pineapple tidbits was a significant reduction in oxidation and desiccation, as best shown in Figure 2. Coated pineapple tidbits kept their color and texture throughout the storage time. The edible coating significantly preserved the fruit's natural juices for a longer period than the control. Also, the natural vanilla essence present in the edible coating enhanced the fruit's sweetness.

The sensory attributes of appearance, texture and taste were evaluated on a scale of 1 to 5, as described above. These three quality attributes were evaluated for the pineapple throughout the storage period of 15 days.

Control tidbits lost their juice and their texture was weakened after 10 days. Appearance and taste scored 4 but texture scored 3 at 10 days of storage. The coated pineapple tidbits scored 4 for appearance, texture and taste at 14 days. It was harder to maintain the initial quality of the pineapple tidbits but coated pieces retained significantly their inherent juices as compared to control.

### EXAMPLE 4

### EFFECTIVENESS OF THE EDIBLE COATING WITH HIGHBUSH BLUEBERRIES

### Blueberries supply

Highbush blueberries were cultivated in Chile and were purchased from Margi-Fruits, Quebec, Canada. Highbush blueberries were carried from Chile to Canada in monitored refrigerated containers in 5 to 10 days.

### Preparation of blueberries

Blueberries were soaked in a peracetic acid and hydrogen peroxide solution (Chinook® or Tsunami 100®) for 15 seconds. The temperature of the solution was kept at 4°C.

### Preparation of sodium alginate coating

Sodium alginate (1.5% w/w) and calcium ascorbate (15% w/w) solutions were prepared as described above.

### Coating of the blueberries with the sodium alginate gel

Blueberries were divided into a control portion and a test portion.

The test blueberries were individually coated in the sodium alginate solution for 10 seconds. Blueberries were held on a conveyor belt and excess solution was drained for 10 seconds.

The blueberries were immersed in the calcium ascorbate solution for 15 to 20 seconds. Once the blueberries had jellified in the calcium ascorbate solution, they were held on a conveyor belt to allow excess solution to drain. Blueberries were later air dried for 8 min with a domestic hair dryer (20-24 km/hr) on a conveyor belt.

### Coated blueberries clusters conditioning

Control blueberries and coated blueberries were later packaged in PETE 3.7 oz plastic trays heat-sealed with a microperforated PET based film, as described above. The headspace within the trays accounts for 50% of the total volume. All sealed containers were refrigerated at 4°C for 21 days.

### Results

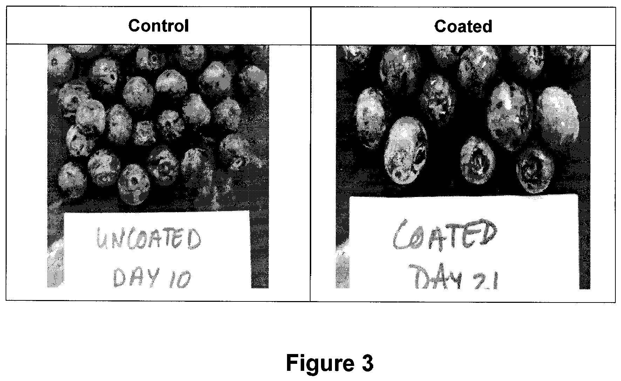

Results are shown in Figure 3. The most noticeable effect of coating was a significant reduction in oxidation and desiccation.

The sensory attributes of appearance, texture and taste were evaluated on a scale of 1 to 5, as described above. These three quality attributes were evaluated for the blueberries throughout the storage period of 21 days.

Control berries became significantly dehydrated after 10 days. Texture and appearance scored a 3 whereas taste scored 4 at 10 days. On the other hand, coated blueberries scored 5 for appearance, texture and taste at 21 days. Thus, the initial quality was preserved by the coating. However, the quality of the berries showed to vary significantly depending from which supplier the blueberries were obtained, which in turn appeared to influence the shelf-life of the coated blueberries.

### EXAMPLE 5

### EFFECTIVENESS OF THE EDIBLE COATING TO FORM CLUSTERS WITH SMALL WHOLE FRUITS (BLUEBERRIES)

### Blueberries supply

Lowbush blueberries from the Lac St-Jean region were purchased from a local market. These blueberries are generally much smaller than imported highbush blueberries. Because they have a thinner peel, lowbush blueberries also tend to be more fragile. Since they are frequently damaged, lowbush blueberries are generally used as ingredients for the manufacture of transformed products (e.g. pies, jams, etc.) rather than being consumed as fresh fruits.

### Preparation of blueberries

Lowbush blueberries were soaked in a peracetic acid and hydrogen peroxide solution (Chinook® or Tsunami 100®) for 15 seconds. The temperature of the solution was kept at 4°C.

### Preparation of sodium alginate coating

Sodium alginate (1.5% w/w) and calcium ascorbate (15% w/w) solutions were prepared as described above.

### Coating of the blueberries with the sodium alginate gel

Blueberries were coated in the sodium alginate solution for 10 seconds and clusters were formed manually by grouping gently handfuls of blueberries. Blueberries clusters were held on a conveyor belt and excess solution was drained for 10 seconds.

The blueberries clusters were then gently immersed in the calcium ascorbate solution for 15 to 20 seconds. Once the blueberries clusters had jellified in the calcium ascorbate solution, they were held on a conveyor belt to allow excess solution to drain. Blueberries were later air dried for 8 min with a domestic hair dryer (20-24 km/hr) on a conveyor belt.

### Coated blueberries clusters conditioning

Blueberries clusters were packaged in PETE 3.7 oz plastic trays heat-sealed with a microperforated PET based film, as described above. The headspace within the trays accounts for 50% of the total volume. All sealed containers were refrigerated at 4°C for 21 days.

### Results

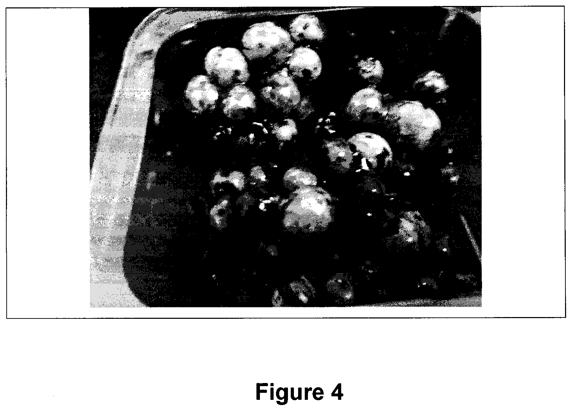

Figure 4 shows the formation of clusters using the method and the edible coating described above.

The use of the edible coating to create blueberry clusters, contributes to prevent mechanical damage associated to handling and transportation. Further, fruit clusters are fun and interactive healthy snacks.

### EXAMPLE 6

### EFFECTIVENESS OF THE EDIBLE COATING IN FORMING CLUSTERS WITH SMALL WHOLE FRUITS OR FRUIT PIECES

### Fresh fruit supply

To determine whether small clusters of coated fruit can be extended to other small fruit products (e.g. pomegranate seeds and fruit pieces), finely chopped apple pieces were tested.

### Apple supply

Gala (Washington, USA) apples were bought from a local supermarket.

### Preparation of apple clusters

Whole apples were soaked in a peracetic acid and hydrogen peroxide solution (Chinook® or Tsunami 100®) for 15 seconds. The temperature of the solution was kept at 4°C. On a sanitized surface, the apples were cored. On a sanitized surface, the apple segments were finely chops in pieces of about 0.5 cm³ using a sanitized sharp knife.

### Preparation of sodium alginate coating

Sodium alginate (1.5% w/w) and calcium ascorbate (15% w/w) solutions were prepared as described above.

### Coating of the apple pieces with the sodium alginate gel

Apple pieces were individually coated in the sodium alginate solution for 10 seconds. Apple pieces were held on a conveyor belt and excess solution was drained for 10 seconds.

Coated apple cluster were then manually formed before immersing the apple cluster in the calcium ascorbate solution for 15 to 20 seconds. Once the apple clusters had jellified in the calcium ascorbate solution, the clusters were held on a conveyor belt to allow excess solution to drain. Apple clusters were later air dried for 8 min with a domestic hair dryer (20-24 km/hr) on a conveyor belt.

Control uncoated chopped apple pieces were packed in the same packaging as coated apple clusters. The apple pieces were previously washed, cored and cut into fine pieces. The control apple pieces were kept in the same controlled atmosphere packaging as the apple clusters in order to compare the relative importance of the edible coating as a mean to extend produce shelf-life.

### Coated apple clusters conditioning

Coated apple clusters were later packaged in PETE 3.7 oz plastic trays heat-sealed with a microperforated PET based film obtained, as described above. The headspace within the trays accounts for 50% of the total volume. All sealed containers were refrigerated at 4°C for 21 days.

### Results

The most noticeable effect of the edible coating on the apple clusters was a significant reduction in oxidation and desiccation. Apple clusters kept their original color and texture throughout the storage time. The edible coating contributed to significantly preserve the natural juices of the apples for a longer period than the control. The natural vanilla essence present in the edible coating enhanced the fruits sweetness.

The sensory attributes of appearance, texture and taste were evaluated on a scale of 1 to 5, as described above. These three quality attributes were evaluated for the apple clusters throughout the storage period of 21 days.

Control apple bits became dehydrated and oxidized within 10 days, rendering a score of 2 for each quality attribute while coated apple clusters maintained initial quality throughout storage time of 21 days. A score of 5 was attributed for appearance, texture and taste.

### EXAMPLE 7

### EFFECTIVENESS OF THE EDIBLE COATING WITH VEGETABLES

### Vegetables supply

Three different mixes of fresh cut vegetables were purchased from a local supplier. The first mix comprises carrots, turnips, onions, and celery. The second mix comprised carrots, onions, celery and bell peppers. The third mix comprised carrots, red onions, celery, leeks, zucchinis and cabbage.

### Preparation of vegetables

Vegetables were soaked in a peracetic acid and hydrogen peroxide solution (Chinook® or Tsunami 100®) for 15 seconds. The temperature of the solution was kept at 4°C.

### Preparation of sodium alginate coating

Sodium alginate (1.5% w/w) and calcium ascorbate (15% w/w) solutions were prepared as described above.

### Coating of the vegetables with the sodium alginate gel

Three vegetable mixes were prepared. The first mix, trivially called the "Soup mix" comprised carrots, turnips, onions (red and yellow) and celery. The second mix, trivially called the "Spaghetti mix" comprised carrots, turnips, onions (red and yellow), celery and peppers. The third mix comprised carrots, turnips, onions (red and yellow), celery and leeks Each vegetable mix was divided into a control portion and a test portion.

The test vegetables were coated in the sodium alginate solution for 10 seconds. Vegetables were held on a conveyor belt and excess solution was drained for 10 seconds.

The vegetables were immersed in the calcium ascorbate solution for 15 to 20 seconds. Once the blueberries had jellified in the calcium ascorbate solution, they were held on a conveyor belt to allow excess solution to drain. Blueberries were later air dried for 8 min with a domestic hair dryer (20-24 km/hr) on a conveyor belt.

### Coated vegetables conditioning

Control vegetables and coated vegetables were later packaged in PETE 3.7 oz plastic trays heat-sealed with a microperforated PET based film, as described above. The headspace within the trays accounts for 50% of the total volume. All sealed containers were refrigerated at 4°C for 21 days.

### Results

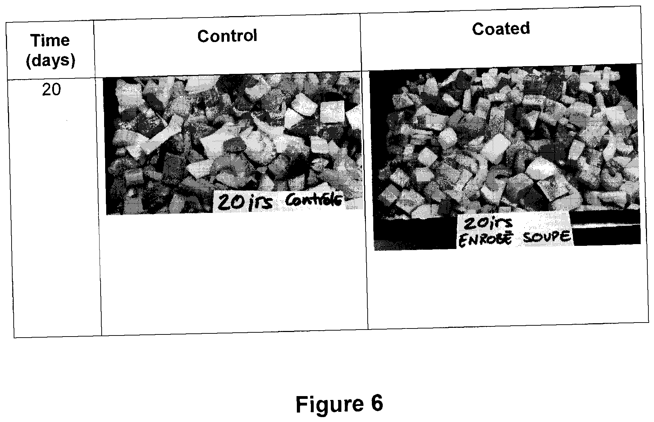

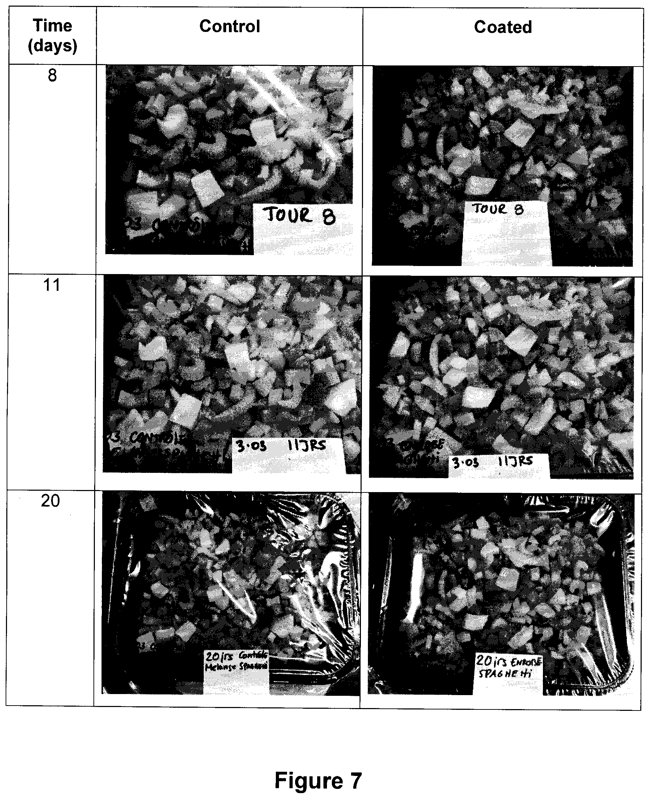

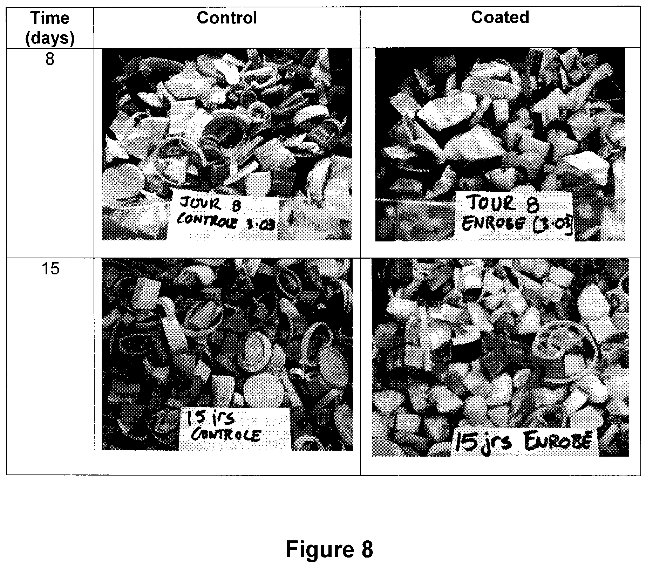

Results are shown in Figure 6 (first mix), Figure 7 (second mix) and Figure 8 (third mix). The most noticeable effect of coating was a significant reduction in oxidation and desiccation. Most of the vegetable displayed a better appearance (*i.e.* color and texture) when coated and the coated vegetables mostly preserved their initial quality. Experiments on vegetables thus showed that the edible coating contributes to extend the shelf-life of vegetables, just as with fruits.

Although the above description relates to a specific embodiment as presently contemplated by the inventor, it will be understood that the discovery described herein in its broad aspect includes mechanical and functional equivalents of the elements described herein.

### Preparation of vegetables

Vegetables were soaked in a peracetic acid and hydrogen peroxide solution (Chinook® or Tsunami 100®) for 15 seconds. The temperature of the solution was kept at 4°C.

### Preparation of sodium alginate coating

Sodium alginate (1.5% w/w) and calcium ascorbate (15% w/w) solutions were prepared as described above.

### Coating of the vegetables with the sodium alginate gel

Three vegetable mixes were prepared. The first mix, trivially called the "Soup mix" comprised carrots, turnips, onions (red and yellow) and celery. The second mix, trivially called the "Spaghetti mix" comprised carrots, turnips, onions (red and yellow), celery and peppers. The third mix comprised carrots, turnips, onions (red and yellow), celery and leeks

Each vegetable mix was divided into a control portion and a test portion.

The test vegetables were coated in the sodium alginate solution for 10 seconds. Vegetables were held on a conveyor belt and excess solution was drained for 10 seconds.

The vegetables were immersed in the calcium ascorbate solution for 15 to 20 seconds. Once the blueberries had jellified in the calcium ascorbate solution, they were held on a conveyor belt to allow excess solution to drain. Blueberries were later air dried for 8 min with a domestic hair dryer (20-24 km/hr) on a conveyor belt.

### Coated vegetables conditioning

Control vegetables and coated vegetables were later packaged in PETE 3.7 oz plastic trays heat-sealed with a microperforated PET based film, as described above. The headspace within the trays accounts for 50% of the total volume. All sealed containers were refrigerated at 4°C for 21 days.

### Results

Results are shown in Figure 6 (first mix), Figure 7 (second mix) and Figure 8 (third mix). The most noticeable effect of coating was a significant reduction in oxidation and desiccation. Most of the vegetable displayed a better appearance (i.e. color and texture) when coated and the coated vegetables mostly preserved their initial quality. Experiments on vegetables thus showed that the edible coating contributes to extend the shelf-life of vegetables, just as with fruits.

## Claims

1. A method for coating a food product with an edible coating, the method comprising:
- coating said food product with a polysaccharide solution to cover said food product, said polysaccharide solution including at least one polysaccharide selected from the group consisting of carrageenan, gellan, alginate and pectin;
- cross-linking said polysaccharide solution by immersing said food product with a cross-linking agent solution to obtain a polysaccharide layer covering said food product, said cross-linking agent solution comprising a calcium ascorbate solution; and
- reducing the moisture content of said polysaccharide layer to obtain said edible coating using a drying process that minimizes thermoshocks to the food product, wherein the drying process is a process selected from the group consisting of a compression process, a desiccation process and a surface lyophilisation process and whereby the moisture content of the polysaccharide layer is reduced by 30% to 97% or, in other words, as such as the moisture content of the edible coating ranges from 3% to 70%.

2. The method according to claim 1, wherein said edible coating has a pH above 3.

3. Method according to claim 1, wherein the polysaccharide solution is a sodium alginate solution and comprises between 0.1% (w/w) and 8% (w/w) sodium alginate.

4. Method according to claim 3, wherein the sodium alginate solution comprises between 0.5% (w/w) and 4% (w/w) sodium alginate.

5. Method according to claim 1, wherein the calcium ascorbate solution comprises between 0.5% (w/w) and 34% (w/w) calcium ascorbate.

6. Method according to claim 5, wherein the calcium ascorbate solution comprises between 13% (w/w) and 27% (w/w) calcium ascorbate.

7. Method according to claim 1, wherein the food product is immersed in the cross-linking agent solution for a period of time ranging from 1 second to 15 minutes.

8. Method according to claim 7, wherein the food product is immersed in the cross-linking agent solution for a period of time ranging, from 10 seconds to 4 minutes.

## Patentansprüche

1. Verfahren zum Beschichten eines Nahrungsmittelprodukts mit einer essbaren Beschichtung, wobei das Verfahren Folgendes umfasst:
- Beschichten des Nahrungsmittelprodukts mit einer Polysaccharidlösung, um das Nahrungsmittelprodukt zu bedecken, wobei die Polysaccharidlösung mindestens ein Polysaccharid einschließt, ausgewählt aus der Gruppe, bestehend aus Carragen, Gellan, Alginat und Pektin;
- Vernetzen der Polysaccharidlösung durch Eintauchen des Nahrungsmittelprodukts in einer Vernetzungsmittellösung, um eine Polysaccharidschicht zu erhalten, die das Nahrungsmittelprodukt abdeckt, wobei die Vernetzungsmittellösung eine Calciumascorbatlösung umfasst; und
- Reduzieren des Feuchtigkeitsgehalts der Polysaccharidschicht, um die essbare Beschichtung zu erhalten, unter Verwendung eines Trocknungsprozesses, der Thermoschocks für das Nahrungsmittelprodukt minimiert, wobei der Trocknungsprozess ein Prozess ist, ausgewählt aus der Gruppe, bestehend aus einem Verdichtungsprozess, einem Austrocknungsprozess und einem Oberflächenlyophilisierungsprozess, und wobei der Feuchtigkeitsgehalt der Polysaccharidschicht um 30 % bis 97 % reduziert wird, oder, mit anderen Worten, so dass der Feuchtigkeitsgehalt der essbaren Beschichtung im Bereich zwischen 3 % und 70 % liegt.

2. Verfahren nach Anspruch 1, wobei die essbare Beschichtung einen pH-Wert von mehr als 3 aufweist.

3. Verfahren nach Anspruch 1, wobei die Polysaccharidlösung eine Natriumalginatlösung ist und zwischen 0,1 % (w/w) und 8 % (w/w) Natriumalginat umfasst.

4. Verfahren nach Anspruch 3, wobei die Natriumalginatlösung zwischen 0,5 % (w/w) und 4 % (w/w) Natriumalginat umfasst.

5. Verfahren nach Anspruch 1, wobei die Calciumascorbatlösung zwischen 0,5 % (w/w) und 34 % (w/w) Calciumascorbat umfasst.

6. Verfahren nach Anspruch 5, wobei die Calciumascorbatlösung zwischen 13 % (w/w) und 27 % (w/w) Calciumascorbat umfasst.

7. Verfahren nach Anspruch 1, wobei das Nahrungsmittelprodukt in die Vernetzungsmittellösung während eines Zeitraums im Bereich zwischen 1 Sekunde und 15 Minuten eingetaucht wird.

8. Verfahren nach Anspruch 7, wobei das Nahrungsmittelprodukt in die Vernetzungsmittellösung während eines Zeitraums im Bereich zwischen 10 Sekunden und 4 Minuten eingetaucht wird.

## Revendications

1. Procédé pour enrober un aliment avec un enrobage comestible, ledit procédé comprenant les étapes suivantes :
- on enrobe ledit aliment avec une solution de polysaccharide pour recouvrir ledit aliment, la solution de polysaccharide comprenant au moins un polysaccharide choisi dans le groupe qui consiste en les carraghénanes, la gomme gellane, l'alginate et la pectine ;
- on réticule ladite solution de polysaccharide en immergeant l'aliment dans une solution d'un agent de réticulation pour obtenir une couche de polysaccharide recouvrant ledit aliment, ladite solution d'agent de réticulation comprenant une solution d'ascorbate de calcium ; et
- on réduit la teneur en humidité de ladite couche de polysaccharide pour obtenir ledit revêtement comestible, en utilisant un procédé de séchage qui limite les chocs thermiques dans l'aliment, où le procédé de séchage est un procédé choisi dans le groupe consistant en un procédé de compression, un procédé de dessiccation et un procédé de lyophilisation en surface et où la teneur en humidité de la couche de polysaccharide est réduite de 30% à 97% ou autrement dit, elle est réduite dans une mesure telle que la teneur en humidité de l'enrobage comestible va de 3% à 70%.

2. Procédé selon la revendication 1, selon lequel l'enrobage comestible à un pH supérieur à 3.

3. Procédé selon la revendication 1, selon lequel la solution de polysaccharide est une solution d'alginate de sodium et comprend entre 0,1% (p/p) et 8% (p/p) d'alginate de sodium.

4. Procédé selon la revendication 3, selon lequel la solution d'alginate de sodium comprend entre 0,5% (p/p) et 4% (p/p) d'alginate de sodium.

5. Procédé selon la revendication 1, selon lequel la solution d'ascorbate de calcium comprend entre 0,5% (p/p) et 34% (p/p) d'ascorbate de calcium.

6. Procédé selon la revendication 5, selon lequel la solution d'ascorbate de calcium comprend entre 13% (p/p) et 27% (p/p) d'ascorbate de calcium.

7. Procédé selon la revendication 1, selon lequel l'aliment est immergé dans la solution d'agent de réticulation durant une période de 1 seconde à 15 minutes.

8. Procédé selon la revendication 7, selon lequel l'aliment est immergé dans la solution d'agent de réticulation durant une période de 10 secondes à 4 minutes.
